# FASCICULE DE BREVET EUROPEEN

(11) **EP 2 789 315 B1**
(45) Date de publication et mention de la délivrance du brevet: **16.03.2016**
(21) Numéro de dépôt: 14160899.2
(22) Date de dépôt: 20.03.2014
(51) Int. Cl.: A61F 2/46, A61F 2/00

(54) **Procédé d'emballage et de conditionnement stérile d'une cupule cotyloïdienne de prothèse de hanche**
Steriles Verpackungsverfahren einer künstlichen Gelenkpfanne einer Hüftprothese
Method for sterile packing and packaging of an acetabular cup for a hip prosthesis

(30) Priorité: 12.04.2013 FR 1353334
(43) Date de publication de la demande: 15.10.2014
(73) Titulaire: Groupe Lepine, 69730 Genay (FR)
(72) Inventeur: Pfaifer, Patrick, 69250 MONTANY (FR); Amorim, Cédric, 69580 SATHONAY CAMP (FR)
(74) Mandataire: Jeannet, Olivier

(56) Documents cités:
- EP-A1- 1 205 165
- EP-A1- 1 698 306
- WO-A1-2012/153092
- FR-A1- 2 877 210

## Description

La présente invention concerne un procédé d'emballage et de conditionnement stérile d'une cupule cotyloïdienne de prothèse de hanche. Elle concerne également un emballage de conditionnement stérile d'une cupule cotyloïdienne, permettant la mise en oeuvre de ce procédé, et un instrument de saisie de la cupule cotyloïdienne, utilisable avec cet emballage.

Un implant cotyloïdien de prothèse de hanche comprend, comme cela est bien connu, une cupule de forme générale sensiblement hémisphérique, destinée à être implantée dans la cavité cotyloïdienne du bassin d'un patient, et un insert en polyéthylène, destiné à être reçu dans la cavité délimitée par la cupule ; cet insert délimite lui-même une cavité articulaire sensiblement hémisphérique, destinée à recevoir la tête d'articulation sphérique d'un implant fémoral.

La cupule est conditionnée dans un emballage stérile tel que celui décrit par exemple dans le document WO2012/153092, qui est ouvert au moment de l'implantation ; pour saisir cette cupule dans cet emballage puis l'implanter dans la cavité cotyloïdienne, il est connu d'utiliser un instrument comprenant une tête de préhension de la cupule et un manche auquel cette tête est apte à être reliée ; la tête de préhension inclut une paroi en dôme adaptée à être engagée dans la cavité de la cupule et un joint périphérique d'étanchéité destiné à venir en application contre la paroi de la cupule dans cette cavité ; la tête de préhension est en outre percée d'un conduit apte à être relié à des moyens à dépression aménagés sur le manche ; ces moyens à dépression permettent, lorsque le joint est appliqué contre la cupule, de réaliser un vide d'air entre la cupule et la tête de préhension, permettant la saisie de la cupule par cette tête. Le manche sert quant à lui à l'impaction de la cupule dans la cavité cotyloïdienne du bassin. Le document FR 2 877 210 divulgue un instrument de ce type. Les documents EP 1205165 et EP 1698306 illustrent également l'état de la technique.

L'expression "vide d'air" sera employée dans la présente description pour signifier un vide d'air ou simplement une dépression d'air n'allant pas jusqu'au vide.

La technique précitée donne globalement satisfaction mais n'est cependant pas dépourvue d'inconvénients. En effet, l'instrument précité a une structure relativement complexe, posant des problèmes de parfaite stérilisation et de résistance du joint d'étanchéité à la répétition des cycles de stérilisation. La détérioration du joint par suite de la répétition des cycles de stérilisation peut avoir une incidence notable sur la réalisation du vide, et donc sur la fiabilité de la saisie de la cupule.

La présente invention vise à remédier à ces inconvénients importants.

À cet effet, le procédé qu'elle concerne comprend les étapes consistant à :
a) prévoir un instrument comprenant un manche et une tête de préhension de la cupule, cette tête de préhension étant séparée de ce manche et apte à être reliée à ce dernier ; la tête de préhension inclut :
   - une paroi ayant un diamètre légèrement inférieur au diamètre de l'ouverture de la cavité de la cupule et présentant une gorge de réception d'un joint périphérique d'étanchéité, ce joint périphérique faisant saillie radialement par rapport à cette paroi, et
   - un rebord périphérique destiné à venir en appui contre la tranche de la cupule délimitant l'ouverture de la cavité de cette cupule ;
   la tête de préhension est apte à être engagée dans la cavité de la cupule selon deux positions, à savoir :
   - une position d'engagement partiel, dans laquelle le joint est simplement en contact par frottements doux avec la paroi de la cupule et ne fait pas obstacle à un passage de l'air entre lui et cette paroi, et dans laquelle ledit rebord périphérique est à distance de la tranche de la cupule ; et
   - une position d'engagement complet, dans laquelle le joint est appliqué contre la paroi de la cupule et fait obstacle à un passage de l'air entre lui et cette paroi, et dans laquelle ledit rebord périphérique d'appui est en appui contre la tranche de la cupule ;
   le diamètre de ladite paroi de la tête de préhension est par ailleurs tel que, dans ladite position d'engagement complet, la tête de préhension soit apte à être basculée par rapport à la cupule de manière à décoller ledit joint de la paroi de la cupule ;
b) insérer la tête de préhension dans la cavité de la cupule, de telle sorte que cette tête soit dans ladite position d'engagement partiel ;
c) insérer cet ensemble tête-cupule, dans cet état, dans un récipient en matériau étanche à l'air ;
d) réaliser un vide d'air dans le récipient de manière à réaliser un vide d'air entre la tête de préhension et la cupule, et
e) sceller le récipient dans cet état de vide d'air.

Le procédé selon l'invention consiste ainsi (i) à concevoir la tête de préhension de manière à ce qu'elle puisse avoir deux positions d'engagement dans la cupule, assez proches l'une de l'autre, l'une, d'engagement partiel dans la cupule, dans laquelle la tête de préhension n'est pas en relation d'étanchéité avec la cupule, et l'autre, déterminée et contrôlée par la venue dudit rebord d'appui contre la tranche de la cupule, d'engagement complet dans cette cupule, dans laquelle la tête de préhension est en relation d'étanchéité avec la cupule ; (ii) à placer cet ensemble tête-cupule, dans ledit état d'engagement partiel, dans un récipient, et (iii) à réaliser un vide d'air dans ce récipient avant de sceller ce récipient.

Par l'expression "assez proches l'une de l'autre", il faut comprendre que les positions d'engagement partiel et d'engagement complet sont distantes l'une de l'autre, selon l'axe de la cupule, de 2 mm ou de moins de 2 mm. De même, par l'expression "diamètre légèrement inférieur", il faut comprendre que ladite paroi a un diamètre inférieur de 2 mm, ou de moins de 2 mm, à celui de la cavité de la cupule au niveau de l'ouverture équatoriale de cette cavité.

La réalisation du vide d'air permet d'évacuer l'air situé entre la tête de préhension et la cupule et d'amener cette tête dans ladite position d'engagement complet. Au moment de l'implantation, l'utilisateur ouvre le récipient ; l'air, lors de son introduction dans ce récipient, vient exercer une pression sur la tête de préhension et réalise la solidarisation de la tête de préhension et de la cupule par le fait du vide d'air piégé entre celles-ci.

L'ensemble tête-cupule est alors apte à être saisi au moyen du manche afin d'introduire la cupule dans la cavité cotyloïdienne. Ce manche peut comprendre une tête proximale d'impaction lui permettant d'être utilisé pour impacter la cupule dans la cavité cotyloïdienne.

Une fois que la cupule est impactée dans cette cavité cotyloïdienne, le manche est apte à être incliné de manière à basculer la tête de préhension par rapport à la cupule et, ce faisant, à décoller le joint de la paroi de la cupule. De l'air peut alors entrer dans l'espace situé entre la cupule et la tête de préhension, permettant ainsi la séparation de la tête de préhension d'avec la cupule et le retrait de cette tête.

Ainsi que cela résulte de ce qui précède, la tête de préhension selon l'invention est dépourvue de conduit débouchant dans sa paroi destinée à être tournée vers le fond de la cupule, à la différence de ce que prévoit la technique antérieure ; elle a donc une structure simplifiée, lui permettant de pouvoir être à usage unique. Le joint d'étanchéité n'a pas à résister à une répétition de cycles de stérilisation, et peut donc également être à usage unique. Le manche n'a pas à être équipé de moyens à dépression, et a donc aussi une structure simple lui permettant d'être à usage unique.

De préférence, au moins une partie de la paroi dudit récipient est souple, et cette partie souple est disposée en regard de la tête de préhension et/ou de la cupule lorsque cette tête et cette cupule sont placées dans ce récipient.

Cette partie de la paroi souple vient ainsi, lors de la réalisation du vide dans le récipient, porter contre la tête de préhension et/ou de la cupule et les presser de manière à maintenir cette tête et cette cupule en position l'une par rapport à l'autre. Cette pression peut être de nature à faire passer la tête de préhension de ladite position d'engagement partiel à ladite position d'engagement complet.

L'ensemble tête-cupule peut, dans cet état, être transporté sans risque de perte du positionnement de la tête par rapport à la cupule.

De préférence, la totalité de la paroi dudit récipient est souple ; ce dernier peut alors être constitué par un sachet d'enveloppement de l'ensemble tête de préhension-cupule. Dans ce cas, pour éliminer tout risque de perforation de ce sachet lorsque la cupule comprend des pointes ou aspérités d'ancrage osseux, le procédé comprend :
l'utilisation, en plus dudit récipient souple, d'un pot en matériau résistant au poinçonnement ;
une étape b1) consistant soit, avant l'étape b) précitée, à placer la cupule dans ce pot, soit, après l'étape b) précitée, à placer l'ensemble tête-cupule dans ce pot ; et
l'étape c) comprend le fait de placer l'ensemble pot-tête-cupule dans ledit récipient.

L'invention concerne également un emballage de conditionnement stérile d'une cupule cotyloïdienne, utilisé pour la mise en oeuvre du procédé précité ; selon l'invention, cet emballage comprend un récipient en un matériau étanche à l'air, apte à être scellé dans un état de vide d'air, ce récipient ayant des dimensions telles qu'il est apte à recevoir l'ensemble formé par la tête de préhension et la cupule, dans ladite position d'engagement partiel de cette tête par rapport à cette cupule.

De préférence, cet emballage comprenant également ledit pot en un matériau résistant au poinçonnement ; ce pot a des dimensions telles qu'il est apte à recevoir l'ensemble formé par la tête de préhension et la cupule, dans ladite position d'engagement partiel de cette tête par rapport à cette cupule, et le récipient a des dimensions telles qu'il est apte à recevoir ce pot contenant cet ensemble.

L'invention concerne en outre un instrument de saisie d'une cupule cotyloïdienne conditionnée au moyen du procédé précité ; cet instrument comprend un manche et une tête de préhension de la cupule, cette tête de préhension étant séparée de ce manche et apte à être reliée à ce dernier ; la tête de préhension inclut :
- une paroi ayant un diamètre légèrement inférieur au diamètre de l'ouverture de la cavité de la cupule et présentant une gorge de réception d'un joint périphérique d'étanchéité, ce joint périphérique faisant saillie par rapport à cette paroi, et
- un rebord périphérique d'appui contre la tranche de la cupule délimitant l'ouverture de cette cupule ;
la tête de préhension est apte à être engagée dans la cavité de la cupule selon deux positions, à savoir :
- une position d'engagement partiel, dans laquelle le joint est simplement en contact par frottements doux avec la paroi de la cupule et ne fait pas obstacle à un passage de l'air entre lui et cette paroi, et dans laquelle ledit rebord périphérique d'appui est à distance de la tranche de la cupule ; et
- une position d'engagement complet, dans laquelle le joint est appliqué contre la paroi de la cupule et fait obstacle à un passage de l'air entre lui et cette paroi, et dans laquelle ledit rebord périphérique d'appui est en appui contre la tranche de la cupule ;
le diamètre de ladite paroi de la tête de préhension est par ailleurs tel que, dans ladite position d'engagement complet, la tête de préhension soit apte à être basculée par rapport à la cupule de manière à décoller ledit joint de la paroi de la cupule.

Ladite paroi de la tête de préhension est de préférence prolongée par une portion en forme de calotte sphérique aplatie, lui permettant de remplir partiellement le volume de la cavité de la cupule en position d'engagement dans cette cupule.

L'invention sera bien comprise, et d'autres caractéristiques et avantages de celle-ci apparaîtront, en référence au dessin schématique annexé, représentant, à titre d'exemple non limitatif, une forme de réalisation préférée d'un instrument de saisie et d'impaction d'une cupule cotyloïdienne et d'un emballage de conditionnement de cette cupule.
La figure 1 est une vue de côté, en coupe transversale, d'une cupule cotyloïdienne de prothèse de hanche, d'une tête de préhension de cette cupule, d'un joint périphérique d'étanchéité dont est équipée cette tête et d'un pot que comprend ledit emballage de conditionnement de la cupule ;
la figure 2 est une vue de ces mêmes éléments, après mise en place de la tête de préhension dans la cupule et mis en place de l'ensemble dans le pot ;
la figure 3 est une vue des éléments selon la figure 2, après leur mise en place dans un sachet d'emballage ;
la figure 4 est une vue des éléments selon la figure 3, après mise du sachet d'emballage sous vide et scellement de ce sachet ;
la figure 5 est une vue desdits éléments, similaire à la figure 2, au cours du retrait hors du pot de l'ensemble formé par la tête de préhension et la cupule, et
la figure 6 est une vue de la cupule après implantation dans une cavité cotyloïdienne, au cours d'un basculement de la tête de préhension qui permet une séparation de cette tête et de cette cupule.

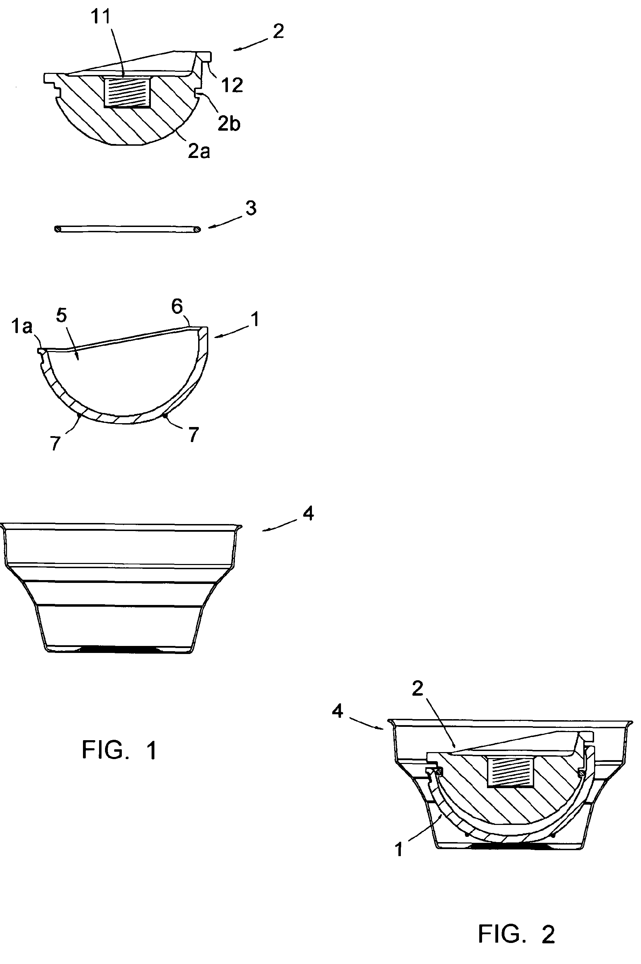

La figure 1 représente : une cupule cotyloïdienne 1 de prothèse de hanche, une tête 2 de préhension de cette cupule 1, un joint périphérique d'étanchéité 3 dont est équipée cette tête 2 et un pot 4 destiné à recevoir la cupule 1 et la tête 2.

La cupule 1 fait partie d'un implant cotyloïdien de prothèse de hanche. Comme cela est bien connu, elle présente une forme générale sensiblement hémisphérique, lui permettant d'être implantée dans la cavité cotyloïdienne du bassin d'un patient, et elle délimite une cavité 5 de réception d'un insert en polyéthylène (non représenté). Cet insert délimite lui-même une cavité articulaire sensiblement hémisphérique, destinée à recevoir la tête d'articulation sphérique d'un implant fémoral.

La cupule 1 et l'insert sont du type bien connu dit "à double mobilité", dans lequel la cupule 1 a une paroi lisse et continue, dépourvue de trous, et la cavité 5 est sensiblement hémisphérique ; l'insert a une face externe de forme générale au moins hémisphérique, apte à glisser de manière multidirectionnelle dans la cavité 5.

Dans l'exemple représenté, la cupule 1 présente un rebord 6 anti-luxation de la tête d'articulation par rapport audit insert, s'étendant sur une majeure partie de la circonférence de la cupule. Cette dernière comprend en outre, du côté de sa zone polaire, des aspérités 7 pour son ancrage à l'os du bassin d'un patient.

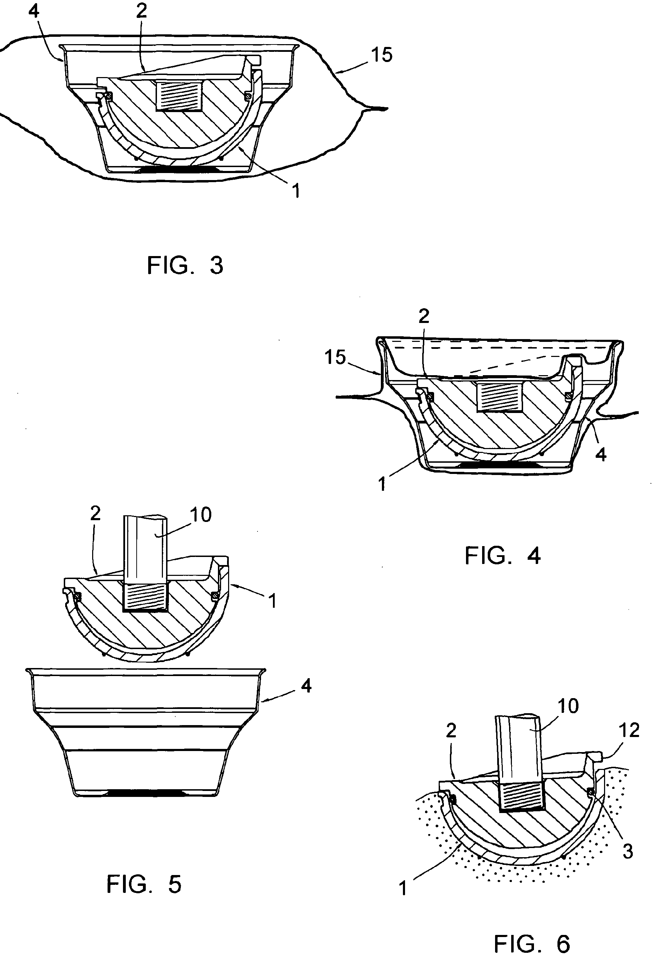

La tête de préhension 2 fait partie, avec le manche 10 visible partiellement sur les figures 5 et 6, d'un instrument de saisie de la cupule 1 dans le pot 4 et d'impaction de cette cupule dans une cavité cotyloïdienne. Elle est destinée à être reliée rigidement à ce manche 10 et comprend à cet effet une cavité centrale taraudée 11 dans laquelle peut être vissée une extrémité filetée du manche 10.

Comme cela est visible sur les figures, la tête de préhension 2 inclut une paroi en forme de calotte sphérique aplatie, dont la base 2a a un diamètre de l'ordre de 1 mm inférieur au diamètre de l'ouverture de la cavité 5 de la cupule 1. Elle présente au niveau de cette base 2a une gorge 2b de réception du joint 3. Cette gorge 2b a, comme représenté, une largeur supérieure à celle de ce joint 3.

En outre, la tête de préhension 2 inclut un rebord périphérique 12 destiné à venir en appui contre la tranche 1a de la cupule 1 qui délimite l'ouverture de la cavité 5. Ce rebord périphérique 12 a une forme adaptée à celle de cette tranche 1 a, qui suit celle du rebord 6 ; le rebord 12 est ainsi apte à venir en appui contre l'ensemble de la tranche 1 a dans la position d'engagement complet de la tête 2 dans la cavité 5 montrée sur les figures 4 et 5.

Le joint 3 est un joint torique s'étendant sur l'ensemble de la circonférence de la tête 2 lorsqu'il est engagé dans la gorge 2b. Dans cet état d'engagement, il fait légèrement saillie radialement par rapport à la base 2a.

Comme le montrent les figures 2 et 4 en comparaison l'une avec l'autre, la tête 2 est apte à être engagée dans la cavité 5 de la cupule 1 selon deux positions, à savoir :
- une position d'engagement partiel montrée sur la figure 2, dans laquelle le joint 3 est simplement en contact par frottements doux avec la paroi de la cupule 1 et le rebord 12 est à distance de la tranche 1a de cette cupule ; dans cette position, le joint 3 ne fait pas obstacle à un passage de l'air entre lui et la paroi de la cupule 1 ; et
- une position d'engagement complet montrée sur la figure 4, dans laquelle le joint 3 est appliqué contre la paroi de la cupule 1 et le rebord 12 est en appui contre la tranche 1 a de cette cupule ; dans cette position, le joint 3 fait obstacle à un passage de l'air entre lui et la paroi de la cupule 1.

Ces deux positions sont distantes l'une de l'autre, selon l'axe de la cupule 1, d'un à deux millimètres environ.

Le pot 4 fait partie d'un emballage de conditionnement de l'ensemble tête 2-cupule 1, comprenant également le sachet 15 visible sur les figures 3 et 4.

Ce pot 4 est dimensionné pour recevoir entièrement ledit ensemble tête 2-cupule 1, comme montré sur la figure 2. Il est en un matériau résistant au poinçonnement, par exemple du PET, de manière à éliminer tout risque de poinçonnement ou perforation lorsqu'il reçoit directement les aspérités 7 contre sa paroi de fond (tel n'est pas le cas dans l'exemple représenté).

Le sachet 15 est en un matériau souple et étanche à l'air, apte à être soudé de manière étanche à l'air ; il peut par exemple s'agir d'un complexe aluminium-matière synthétique.

En référence aux figures 2 à 4 successivement, la tête 2, le pot 4 et le sachet 15 permettent la mise en oeuvre du procédé comprenant les étapes consistant à :
- insérer la tête 2 dans la cavité 5 de la cupule 1, de telle sorte que cette tête 2 soit dans ladite position d'engagement partiel ; les frottements doux précités, conjointement à l'air plus ou moins piégé entre la tête 2 et la cupule 1, permet le maintien de la tête 2 dans cette position d'engagement partiel ;
- insérer cet ensemble tête 2-cupule 1 dans le pot 4 (cf. figure 2) ;
- insérer l'ensemble tête 2-cupule 1-pot 4 dans le sachet 15 (cf. figure 3) ;
- réaliser un vide d'air dans le sachet 15 de manière à réaliser un vide d'air entre la tête 2 et la cupule 1 et à rétracter le sachet 15 contre la tête 2 ;
- sceller le sachet 15 dans cet état de vide d'air (cf. figure 4).

La réalisation du vide d'air permet d'évacuer l'air situé entre la tête 2 et la cupule 1 et d'amener cette tête dans ladite position d'engagement complet. La paroi souple du sachet 15 se rétracte contre la tête 2 et vient ainsi presser cette tête contre la cupule 1. Cette pression permet maintenir cette tête et cette cupule en position l'une par rapport à l'autre et réalise en outre le passage de la tête 2 de ladite position d'engagement partiel à ladite position d'engagement complet.

Au moment de l'implantation de la cupule 1, l'utilisateur perfore le sachet 15 ; l'air, lors de son introduction dans ce sachet, vient exercer une pression sur la tête 2 et réalise la solidarisation de cette tête à la cupule 1 par le fait du vide d'air piégé entre celles-ci.

L'ensemble tête 2-cupule 1 est alors apte à être saisi au moyen du manche 10 (figure 5) afin d'introduire la cupule 1 dans la cavité cotyloïdienne. Le manche 10 comporte une tête d'impaction lui permettant d'impacter la cupule 1 dans la cavité cotyloïdienne.

Lorsque la cupule 1 est impactée dans cette cavité cotyloïdienne, le manche 10 est incliné par rapport à la cupule 1 (figure 6), ce qui a pour effet de décoller le joint 3 de la paroi de la cupule 1 et de permettre à l'air d'entrer dans l'espace situé entre cette cupule et la tête 2. La séparation de cette cupule et de cette tête, ainsi que le retrait de la tête, sont ainsi rendus possibles.

Comme cela apparaît de ce qui précède, l'invention fournit ainsi un procédé d'emballage et de conditionnement stérile d'une cupule cotyloïdienne de prothèse de hanche, un emballage de conditionnement de cette cupule cotyloïdienne, et un instrument de saisie de la cupule cotyloïdienne, qui procurent des avantages déterminants, précités, par rapport à la technique antérieure.

L'invention a été décrite ci-dessus en référence à une forme de réalisation donnée à titre d'exemple. Il va de soi qu'elle n'est pas limitée à cette forme de réalisation mais qu'elle s'étend à toutes les formes de réalisation couvertes par les revendications ci-annexées.

## Revendications

1. Procédé d'emballage et de conditionnement stérile d'une cupule cotyloïdienne (1) de prothèse de hanche, **caractérisé en ce qu'**il comprend les étapes consistant à :
a) prévoir un instrument comprenant un manche (10) et une tête (2) de préhension de la cupule, cette tête de préhension étant séparée de ce manche (10) et apte à être reliée à ce dernier ; la tête de préhension (2) inclut :
- une paroi (2a) ayant un diamètre légèrement inférieur au diamètre de l'ouverture de la cavité de la cupule et présentant une gorge (2b) de réception d'un joint périphérique d'étanchéité (3), ce joint faisant saillie radialement par rapport à cette paroi (2a), et
- un rebord périphérique (12) destiné à venir en appui contre la tranche (1 a) de la cupule (1) délimitant l'ouverture de la cavité (5) de cette cupule ;
la tête de préhension (2) est apte à être engagée dans la cavité (5) de la cupule (1) selon deux positions, à savoir :
- une position d'engagement partiel, dans laquelle le joint (3) est simplement en contact par frottements doux avec la paroi de la cupule (1) et ne fait pas obstacle à un passage de l'air entre lui et cette paroi, et dans laquelle ledit rebord périphérique (12) est à distance de la tranche (1 a) de la cupule (1) ; et
- une position d'engagement complet, dans laquelle le joint (3) est appliqué contre la paroi de la cupule (1) et fait obstacle à un passage de l'air entre lui et cette paroi, et dans laquelle ledit rebord périphérique d'appui (12) est en appui contre la tranche (1a) de la cupule (1) ;
le diamètre de ladite paroi (2a) de la tête de préhension est tel que, dans ladite position d'engagement complet, la tête de préhension (2) soit apte à être basculée par rapport à la cupule (1) de manière à décoller ledit joint (3) de la paroi de la cupule (1) ;
b) insérer la tête de préhension (2) dans la cavité (5) de la cupule (1), de telle sorte que cette tête soit dans ladite position d'engagement partiel ;
c) insérer cet ensemble tête (2)-cupule (1), dans cet état, dans un récipient (15) en matériau étanche à l'air ;
d) réaliser un vide d'air dans le récipient (15) de manière à réaliser un vide d'air entre la tête de préhension (2) et la cupule (1), et
e) sceller le récipient (15) dans cet état de vide d'air.

2. Procédé selon la revendication 1, **caractérisé en ce que** lesdites positions d'engagement partiel et d'engagement complet sont distantes l'une de l'autre, selon l'axe de la cupule, de 2 mm ou de moins de 2 mm.

3. Procédé selon la revendication 1 ou la revendication 2, **caractérisé en ce que** la paroi (2a) a un diamètre inférieur de 2 mm, ou de moins de 2 mm, à celui de la cavité (5) de la cupule (1) au niveau de l'ouverture équatoriale de cette cavité.

4. Procédé selon l'une des revendications 1 à 3, **caractérisé en ce qu'**au moins une partie de la paroi dudit récipient (15) est souple, et **en ce que** cette partie souple est disposée en regard de la tête de préhension (2) et/ou de la cupule (1) lorsque cette tête et cette cupule sont placées dans ce récipient.

5. Procédé selon la revendication 4, **caractérisé en ce que** :
la totalité de la paroi dudit récipient (15) est souple ; et **en ce que**
le procédé comprend :
l'utilisation, en plus dudit récipient souple (15), d'un pot (4) en matériau résistant au poinçonnement ;
une étape b1) consistant soit, avant l'étape b) précitée, à placer la cupule (1) dans ce pot (4), soit, après l'étape b) précitée, à placer l'ensemble tête (2)-cupule (1) dans ce pot (4) ; et
l'étape c) comprend le fait de placer l'ensemble pot (4)-tête (2)-cupule (1) dans ledit récipient (15).

6. Emballage de conditionnement stérile d'une cupule cotyloïdienne (1), utilisé pour la mise en oeuvre du procédé selon l'une des revendications 1 à 5, **caractérisé en ce qu'**il comprend un récipient (15) en un matériau étanche à l'air, apte à être scellé dans un état de vide d'air, ce récipient ayant des dimensions telles qu'il est apte à recevoir l'ensemble formé par la tête de préhension (2) et la cupule (1), dans ladite position d'engagement partiel de cette tête par rapport à cette cupule.

7. Emballage selon la revendication 6, **caractérisé :**
**en ce qu'**il comprend un pot (4) en un matériau résistant au poinçonnement, ayant des dimensions telles qu'il est apte à recevoir l'ensemble formé par la tête de préhension (2) et la cupule (1), dans ladite position d'engagement partiel de cette tête par rapport à cette cupule, et
**en ce que** le récipient (15) a des dimensions telles qu'il est apte à recevoir ce pot (4) contenant ledit ensemble.

8. Instrument de saisie d'une cupule cotyloïdienne (1) conditionnée au moyen du procédé selon l'une des revendications 1 à 5, **caractérisé en ce qu'**il comprend un manche (10) et une tête de préhension (2) de la cupule (1), cette tête de préhension (2) étant séparée de ce manche (10) et apte à être reliée à ce dernier ; la tête de préhension (2) inclut :
- une paroi (2a) ayant un diamètre légèrement inférieur au diamètre de l'ouverture de la cavité de la cupule et présentant une gorge (2b) de réception d'un joint périphérique d'étanchéité (3), ce joint faisant saillie radialement par rapport à cette paroi (2a), et
- un rebord périphérique (12) d'appui contre la tranche (1a) de la cupule (1) délimitant l'ouverture de la cavité (5) de cette cupule ;
la tête de préhension (2) est apte à être engagée dans la cavité (5) de la cupule (1) selon deux positions, à savoir :
- une position d'engagement partiel, dans laquelle le joint (3) est simplement en contact par frottements doux avec la paroi de la cupule (1) et ne fait pas obstacle à un passage de l'air entre lui et cette paroi, et dans laquelle ledit rebord périphérique d'appui (12) est à distance de la tranche (1a) de la cupule (1) ; et
- une position d'engagement complet, dans laquelle le joint (3) est appliqué contre la paroi de la cupule (1) et fait obstacle à un passage de l'air entre lui et cette paroi, et dans laquelle ledit rebord périphérique d'appui (12) est en appui contre la tranche (1a) de la cupule (1) ;
le diamètre de ladite paroi (2a) de la tête de préhension est tel que, dans ladite position d'engagement complet, la tête de préhension (2) soit apte à être basculée par rapport à la cupule (1) de manière à décoller ledit joint (3) de la paroi de la cupule (1).

9. Instrument selon la revendication 8, **caractérisé en ce que** le manche (10) comprend une tête proximale d'impaction.

10. Instrument selon la revendication 8 ou la revendication 9, **caractérisé en ce que** ladite paroi (2a) est prolongée par une portion en forme de calotte sphérique aplatie, lui permettant de remplir partiellement le volume de la cavité (5) de la cupule (1) en position d'engagement dans cette cupule.

## Patentansprüche

1. Verfahren zur sterilen Verpackung einer künstlichen Gelenkpfanne (1) einer Hüftprothese, **dadurch gekennzeichnet, dass** es die folgenden Schritte umfasst:
a) Vorsehen eines Instruments, das einen Stiel (10) und einen Kopf (2) zum Greifen der Pfanne umfasst, wobei dieser Greifkopf von diesem Stiel (10) getrennt ist und geeignet ist, mit diesem letzteren verbunden zu werden, wobei der Greifkopf (2) Folgendes umfasst:
- eine Wand (2a), die einen Durchmesser aufweist, der etwas kleiner als der Durchmesser der Öffnung des Hohlraums der Pfanne ist und eine Nut (2b) für die Aufnahme einer umlaufenden Dichtung (3) aufweist, wobei diese Dichtung radial in Bezug zu dieser Wand (2a) hervorsteht, und
- einen umlaufenden Flansch (12), der dazu bestimmt ist, gegen den Rand (1 a) der Pfanne (1) in Auflage zu kommen, der die Öffnung des Hohlraums (5) dieser Pfanne abgrenzt;
wobei der Greifkopf (2) geeignet ist, in den Hohlraum (5) der Pfanne (1) in zwei Stellungen in Eingriff gebracht zu werden, und zwar:
- in einer partiellen Eingriffsstellung, in der die Dichtung (3) einfach durch sanfte Reibungen mit der Wand der Pfanne (1) in Berührung ist und einen Durchgang der Luft zwischen ihr und dieser Wand nicht behindert und in der der umlaufende Flansch (12) von dem Rand (1 a) der Pfanne (1) beabstandet ist; und
- in einer vollständigen Eingriffsstellung, in der die Dichtung (3) gegen die Wand der Pfanne (1) angesetzt ist und einen Durchgang der Luft zwischen ihr und dieser Wand behindert und in der der umlaufende Auflageflansch (12) gegen den Rand (1a) der Pfanne (1) in Auflage ist;
wobei der Durchmesser der Wand (2a) des Greifkopfes derart ist, dass in der vollständigen Eingriffsstellung der Greifkopf (2) geeignet ist, in Bezug zu der Pfanne (1) derart gekippt zu werden, dass die Dichtung (3) von der Wand der Pfanne (1) abgelöst wird;
b) Einsetzen des Greifkopfes (2) in den Hohlraum (5) der Pfanne (1), derart, dass dieser Kopf sich in der partiellen Eingriffsstellung befindet;
c) Einsetzen dieser Baugruppe aus Kopf (2) und Pfanne (1) in diesem Zustand in einen Behälter (15) aus luftdichtem Material;
d) Herstellen eines Vakuums in dem Behälter (15), derart, dass ein Vakuum zwischen dem Greifkopf (2) und der Pfanne (1) hergestellt wird, und
e) Versiegeln des Behälters (15) in diesem Vakuumzustand

2. Verfahren nach Anspruch 1, **dadurch gekennzeichnet, dass** die partielle und die vollständige Eingriffsstellung entlang der Achse der Pfanne um 2 mm oder um weniger als 2 mm voneinander beabstandet sind.

3. Verfahren nach Anspruch 1 oder Anspruch 2, **dadurch gekennzeichnet, dass** die Wand (2a) einen Durchmesser aufweist, der um 2 mm oder um weniger als 2 mm kleiner ist als derjenige des Hohlraums (5) der Pfanne (1) im Bereich der äquatorialen Öffnung dieses Hohlraums.

4. Verfahren nach einem der Ansprüche 1 bis 3, **dadurch gekennzeichnet, dass** zumindest ein Teil der Wand des Behälters (15) biegsam ist, und dadurch, dass dieser biegsame Teil dem Greifkopf (2) und/oder der Pfanne (1) gegenüberstehend angeordnet ist, wenn dieser Kopf und diese Pfanne in diesem Behälter platziert werden.

5. Verfahren nach Anspruch 4, **dadurch gekennzeichnet, dass**:
die gesamte Wand des Behälters (15) biegsam ist; und dadurch, dass das Verfahren Folgendes umfasst:
die Verwendung eines Topfes (4) aus stichfestem Material zusätzlich zu dem biegsamen Behälter (15);
einen Schritt b1), der darin besteht, entweder vor dem vorhergehend genannten Schritt b) die Pfanne (1) in diesem Topf (4) zu platzieren oder nach dem vorhergehend genannten Schritt b) die Baugruppe aus Kopf (2) und Pfanne (1) in diesem Topf (4) zu platzieren; und
wobei der Schritt c) die Tatsache umfasst, dass die Baugruppe aus Topf (4), Kopf (2) und Pfanne (1) in dem Behälter (15) platziert wird.

6. Sterile Verpackung einer künstlichen Gelenkpfanne (1), die zur Durchführung des Verfahrens nach einem der Ansprüche 1 bis 5 verwendet wird, **dadurch gekennzeichnet, dass** sie einen Behälter (15) aus einem luftdichten Material umfasst, der geeignet ist, in einem Vakuumzustand versiegelt zu werden, wobei dieser Behälter Abmessungen aufweist, die derart sind, dass er geeignet ist, die Baugruppe, die durch den Greifkopf (2) und die Pfanne (1) gebildet ist, in der partiellen Eingriffsstellung dieses Kopfs in Bezug zu dieser Pfanne aufzunehmen.

7. Verpackung nach Anspruch 6, **dadurch gekennzeichnet, dass**:
sie einen Topf (4) aus einem stichfesten Material umfasst, der Abmessungen aufweist, die derart sind, dass er geeignet ist, die Baugruppe, die durch den Greifkopf (2) und die Pfanne (1) gebildet wird, in der partiellen Eingriffsstellung dieses Kopfs in Bezug zu dieser Pfanne aufzunehmen und
dadurch, dass der Behälter (15) Abmessungen aufweist, die derart sind, dass er geeignet ist, diesen Topf (4), der die Baugruppe enthält, aufzunehmen.

8. Instrument zum Ergreifen einer künstlichen Gelenkpfanne (1), die mittels des Verfahrens nach einem der Ansprüche 1 bis 5 verpackt ist, **dadurch gekennzeichnet, dass** es einen Stiel (10) und einen Kopf (2) zum Greifen der Pfanne (1) umfasst, wobei dieser Greifkopf (2) von diesem Stiel (10) getrennt ist und geeignet ist, mit diesem letzteren verbunden zu werden, wobei der Greifkopf (2) Folgendes umfasst:
- eine Wand (2a), die einen Durchmesser aufweist, der etwas kleiner als der Durchmesser der Öffnung des Hohlraums der Pfanne ist und eine Nut (2b) für die Aufnahme einer umlaufenden Dichtung (3) aufweist, wobei diese Dichtung radial in Bezug zu dieser Wand (2a) hervorsteht, und
- einen umlaufenden Flansch (12) zur Auflage gegen den Rand (1a) der Pfanne (1), der die Öffnung des Hohlraums (5) dieser Pfanne abgrenzt;
wobei der Greifkopf (2) geeignet ist, in den Hohlraum (5) der Pfanne (1) in zwei Stellungen in Eingriff gebracht zu werden, und zwar:
- in einer partiellen Eingriffsstellung, in der die Dichtung (3) einfach durch sanfte Reibungen mit der Wand der Pfanne (1) in Berührung ist und einen Durchgang der Luft zwischen ihr und dieser Wand nicht behindert und in der der umlaufende Auflageflansch (12) von dem Rand (1 a) der Pfanne (1) beabstandet ist; und
- in einer vollständigen Eingriffsstellung, in der die Dichtung (3) gegen die Wand der Pfanne (1) angesetzt ist und einen Durchgang der Luft zwischen ihr und dieser Wand behindert und in der der umlaufende Auflageflansch (12) gegen den Rand (1a) der Pfanne (1) in Auflage ist;
wobei der Durchmesser der Wand (2a) des Greifkopfes derart ist, dass in der vollständigen Eingriffsstellung der Greifkopf (2) geeignet ist, in Bezug zu der Pfanne (1) derart gekippt zu werden, dass die Dichtung (3) von der Wand der Pfanne (1) abgelöst wird.

9. Instrument nach Anspruch 8, **dadurch gekennzeichnet, dass** der Griff (10) einen proximalen Impaktierkopf umfasst.

10. Instrument nach Anspruch 8 oder Anspruch 9, **dadurch gekennzeichnet, dass** die Wand (2a) durch einen Abschnitt mit der Form einer abgeflachten kugelförmigen Haube verlängert ist, der es ihr ermöglicht, das Volumen des Hohlraums (5) der Pfanne (1) in der Eingriffsstellung dieser Pfanne teilweise zu füllen.

## Claims

1. Method for sterile packing and packaging of an acetabular cup for a hip prosthesis, **characterized in that** it comprises the steps of:
a) providing a tool comprising a handle (10) and a gripping head (2) for gripping the cup, the gripping head being separated from said handle (10) and adapted to be connected thereto; the gripping head (2) includes:
- a wall (2a) having a diameter slightly smaller than the diameter of the opening of the cavity of the cup and having a groove (2b) for receiving a peripheral seal (3), this seal projecting radially relative to said wall (2a), and
- a peripheral rim (12) intended to bear against the edge (1 a) of the cup (1) defining the opening of the cavity (5) of the cup;
the gripping head (2) is adapted to be engaged in the cavity (5) of the cup (1) in two positions, namely:
- a partially engaged position in which the seal (3) is simply in contact with the wall of the cup (1) with low friction and does not prevent a passage of air between it and the wall, and wherein said peripheral rim (12) is spaced from the edge (1a) of the cup (1); and
- a fully engaged position, in which the seal (3) is applied against the wall of the cup (1) and preclude the passage of air between itself and said wall, and in which said peripheral bearing rim (12) bears against the edge (1 a) of the cup (1);
the diameter of said wall (2a) of the gripping head is such that, in said fully engaged position, the gripping head (2) is adapted to be tilted relative to the cup (1) so as to unstick said joint (3) from the wall of the cup (1);
b) inserting the gripping head (2) in the cavity (5) of the cup (1), so that this head is in said partially engaged position;
c) inserting the assembly head (2)-cup (1), in this state, in a container (15) made in airtight material;
d) performing a vacuum in the container (15) so as to produce an air vacuum between the gripping head (2) and the cup (1), and
e) sealing the container (15) in this state of air vacuum.

2. Method according to claim 1, **characterized in that** said partially engaged and fully engaged positions are spaced from each other along the axis of the cup, of 2 mm or less than 2 mm.

3. Method according to claim 1 or claim 2, **characterized in that** the wall (2a) has a diameter which is of 2 mm, or less than 2 mm, less than that of the cavity (5) of the cup (1) at the equatorial opening of said cavity.

4. Method according to one of claims 1 to 3, **characterized in that** at least a portion of the wall of said container (15) is flexible, and **in that** said flexible portion is disposed opposite the gripping head (2) and / or the cup (1) when this head and the cup are placed into this container.

5. Method according to claim 4, **characterized in that**:
the entire wall of said container (15) is flexible; and **in that**
the method comprises:
the use, in addition to said flexible container (15), of a pot (4) made in a puncture resistant material;
a step b1) consisting, either before step b) above, to place the cup (1) in the pot (4), or after step b) above, to place the head assembly (2) -cupule (1) into the pot (4); and
step c) comprises placing the assembly pot (4)-head (2)-cup (1) in said container (15).

6. Packing for sterile packaging of an acetabular cup (1), used for implementing the method according to one of claims 1 to 5, **characterized in that** it comprises a container (15) made in an airtight material, sealable in an air vacuum condition, this container having dimensions such that it is capable of receiving the assembly formed by the gripping head (2) and the cup (1), in said partially engaged position of the head relative to the cup.

7. Packaging according to claim 6, **characterized:**
**in that** it comprises a pot (4) made in a puncturing resistant material, having dimensions such that it is capable of receiving the assembly formed by the gripping head (2) and the cup (1), in said partially engaged position of the head relative to the cup, and
**in that** the container (15) has dimensions such that it is adapted for receiving the pot (4) containing said assembly.

8. Instrument for gripping an acetabular cup (1) conditioned by the method according to one of claims 1 to 5, **characterized in that** it comprises a handle (10) and a gripping head (2) for gripping the cup (1), said gripping head (2) being separated from the handle (10) and adapted to be connected thereto; the gripping head (2) includes:
- a wall (2a) having a diameter slightly smaller than the diameter of the opening of the cavity of the cup and having a groove (2b) for receiving a peripheral seal (3), this seal projecting radially relative to said wall (2a), and
- a peripheral rim (12) bearing against the edge (1 a) of the cup (1) defining the opening of the cavity (5) of the cup;
the gripping head (2) is adapted to be engaged in the cavity (5) of the cup (1) in two positions, namely:
- a partially engaged position in which the seal (3) is simply in contact with the wall of the cup (1) with low friction and does not preclude the passage of air between it and the wall, and in which said peripheral bearing rim (12) is spaced from the edge (1 a) of the cup (1); and
- a fully engaged position, in which the seal (3) is applied against the wall of the cup (1) and preclude the passage of air between itself and said wall, and in which said peripheral bearing rim (12) bears against the edge (1 a) of the cup (1);
the diameter of said wall (2a) of the gripping head is such that in said fully engaged position, the gripping head (2) is adapted to be tilted relative to the cup (1) so as to unstick said joint (3) from the wall of the cup (1).

9. Instrument as claimed in claim 8, **characterized in that** the handle (10) includes a proximal impaction head.

10. Instrument as claimed in claim 8 or claim 9, **characterized in that** said wall (2a) is extended by a portion in the form of flattened spherical cup, allowing it to partially fill the volume of the cavity (5) of the cup (1) in the engagement position in this cup.
